# FASCICULE DE BREVET EUROPEEN

(11) **EP 2 358 455 B1**
(45) Date de publication et mention de la délivrance du brevet: **24.07.2019**
(21) Numéro de dépôt: 09756509.7
(22) Date de dépôt: 24.11.2009
(51) Int. Cl.: B01D 9/00, C07C 51/43, C07C 55/14

(54) **INSTALLATION DE CRISTALLISATION D'ACIDE ADIPIQUE**
EINRICHTUNG ZUM KRISTALLISIEREN VON ADIPINSÄURE
INSTALLATION FOR CRYSTALLISING ADIPIC ACID

(30) Priorité: 01.12.2008 FR 0806729
(43) Date de publication de la demande: 24.08.2011
(73) Titulaire: Rhodia Opérations, 93306 Aubervilliers (FR)
(72) Inventeur: CARVIN, Philippe, F-69005 Lyon (FR); BELLENGER, Fabien, 69230 Saint Genis Laval (FR); CROTTIER-COMBE, Serge, F-38790 Diemoz (FR)
(74) Mandataire: Schuck, Alexander
(86) Numéro de dépôt international: PCT/EP2009/065758
(87) Numéro de publication internationale: WO 2010/063619

(56) Documents cités:
- US-A- 4 014 903
- TSINMAN A I ET AL: "Corrosion of stainless steels in fused dicarboxylic acids" JOURNAL OF APPLIED CHEMISTRY OF USSR, CONSULTANTS BUREAU, NEW YORK, NY, US, vol. 38, no. 8, 1 août 1965 (1965-08-01), pages 1828-1831, XP009116445 ISSN: 0021-888X
- KACHANOV V A ET AL: "CORROSION OF WELD JOINTS IN THE MANUFACTURE OF ADIPIC ACID" PROTECTION OF METALS, PLENUM PUBLISHING CO, NEW YORK, US, vol. 17, no. 6, 1 novembre 1981 (1981-11-01), pages 599-602, XP009116448 ISSN: 0033-1732
- % Composition ET AL: "Corrosion Resistance of Nickel-Containing Alloys in Organic Acids and Related Compounds Martensitic & Ferritic Stainless Steels 15 D. Duplex Austenitic-Ferritic and Precipitation Hardening Stainless Steels 15 E. Iron-Base Nickel-Chromium-Copper Molybdenum Alloys 16 F. Nickel-Base Chromium-Iron-Molyb", Fatty Acids Tall Oil Acids) E. Di and Tricarboxylic Acids Balance Balance, 1 January 1979 (1979-01-01), pages 25-0, XP055258039, Retrieved from the Internet: URL:https://www.nickelinstitute.org/~/medi a/Files/TechnicalLiterature/CorrosionResis tanceofNickel_ContainingAlloysinOrganicAci dsandRelatedCompounds_1285_.ashx [retrieved on 2016-03-14]

## Description

La présente invention concerne une installation de cristallisation d'acide adipique.

L'acide adipique est un composé chimique important utilisé comme matière première dans la synthèse de nombreux composés. Ainsi, l'acide adipique est un composé intermédiaire important dans la synthèse des polyamides et plus particulièrement du PA 6.6., ainsi que dans la fabrication des polyesters, polyuréthannes. L'acide adipique est également utilisé comme additif dans de nombreuses autres applications comme la fabrication de plastifiants, par exemple.

L'acide adipique est généralement synthétisé à partir du cyclohexane, par oxydation de celui-ci en un mélange cyclohexanone/cyclohexanol puis oxydation nitrique de ce mélange en acide adipique. Plusieurs procédés d'oxydation du cyclohexane en cyclohexanone/cyclohexanol sont mis en oeuvre avec des catalyseurs différents.

L'oxydation nitrique du mélange cyclohexanol/cyclohexanone est réalisée en présence de catalyseur métallique, l'acide adipique étant généralement récupéré et purifié par cristallisations successives.

Il a également été proposé des procédés d'oxydation directe du cyclohexane en acide adipique par l'oxygène ou l'air, en présence de catalyseurs métalliques et d'un solvant tel que l'acide acétique. Dans ces procédés, l'acide adipique est généralement récupéré sous forme de solution aqueuse.

Quel que soit le procédé de synthèse de l'acide adipique, il est nécessaire pour obtenir un composé compatible pour notamment les utilisations décrites ci-dessus, de le purifier. Les procédés de purification généralement utilisés mettent en oeuvre des étapes de cristallisation (comme décrit dans le document US 4,014,903) consistant classiquement à concentrer et/ou à refroidir la solution d'acide adipique pour obtenir la formation de cristaux d'acide adipique pur.

Ces cristallisations sont mises en oeuvre généralement dans des cristallisoirs de grandes dimensions. Une installation de cristallisation peut comprendre plusieurs cristallisoirs montés en série ou un appareil comprenant plusieurs compartiments permettant de réaliser une purification en continu ou plusieurs cristallisoirs fonctionnant selon un procédé discontinu.

Les cristallisoirs sont généralement équipés de moyens d'agitation, de moyens pour refroidir et/ou concentrer la solution. Ces derniers moyens peuvent être constitués par des dispositifs mis en contact avec la solution et dans lesquels circule un fluide caloporteur, des moyens permettant d'évaporer la solution notamment par mise sous pression réduite.

Les moyens pour refroidir et/ou concentrer la solution peuvent comprendre plusieurs dispositifs utilisés seuls ou en combinaison, comme, par exemple, une double enveloppe dans la paroi du cristallisoir, des éléments comprenant des moyens pour faire circuler un fluide caloporteur disposés dans la solution ou un circuit externe de circulation de la solution comprenant un échangeur de chaleur. Cette liste de moyens pour refroidir et/ou concentrer la solution n'est donnée qu'à titre indicatif et n'a pas de caractère limitatif.

Pour obtenir un fonctionnement correct du cristallisoir et maintenir sa productivité, il est nécessaire de contrôler certains paramètres de fonctionnement et plus particulièrement les paramètres régissant les phénomènes d'encrassement du cristallisoir par dépôt d'acide adipique cristallisé sur les parois de l'appareillage. Ce phénomène appelé « blindage » dépend de la nature du matériau et de l'état de surface des parois. Plus particulièrement, il sera favorisé par la différence de température entre la solution et les parois en contact avec la solution. En effet, si cette différence de température est supérieure à une valeur critique fonction de la concentration et de la température de la solution, du débit de circulation de la solution dans le cristallisoir, de la nature du matériau et état de surface de la paroi, il se produit un dépôt d'acide adipique qui adhère à la paroi.

Le dépôt d'acide adipique est également observé, indépendamment de la différence de température sur les parois dont l'état de surface est dégradé, notamment dans le cas d'une cristallisation par concentration de la solution obtenue par mise sous pression réduite du cristallisoir.

Pour retrouver des conditions acceptables de fonctionnement du cristallisoir, il est nécessaire d'arrêter périodiquement le procédé pour éliminer les dépôts d'acide adipique sur les parois d'échange de chaleur ou les parois du cristallisoir.

De plus, un décollement intempestif de ce dépôt au cours d'une opération de production d'acide adipique peut entrainer des dégâts mécaniques, voir induire une fluctuation de la qualité de l'acide adipique produit.

Pour limiter ce phénomène de blindage, les parois internes du cristallisoir ainsi que les parois du dispositif pour refroidir et/ou concentrer la solution sont polies pour obtenir un état de surface avec une rugosité minimale. Ces surfaces polies peuvent également être nettoyées et lavées par les techniques classiques de nettoyage utilisées dans le domaine de traitement des surfaces métalliques.

Toutefois, l'état de surface des parois peut se dégrader rapidement sous l'effet de la corrosion chimique notamment quand la cristallisation de l'acide adipique est réalisée à partir des solutions obtenues lors de l'oxydation nitrique des mélanges cyclohexanone/cyclohexanol. En effet, ces solutions sont acides et contiennent une quantité importante d'acide nitrique et/ou d'ions nitrates.

Pour limiter le phénomène de blindage et éventuellement la dégradation de l'état de surface, les cristallisoirs et les parois des dispositifs d'échange sont souvent réalisés en acier inoxydable austénitique de type AISI 304L. Toutefois, ces phénomènes de blindage et corrosion sont toujours constatés. L'utilisation de ce type de matériau ne permet pas de supprimer ou diminuer les arrêts de l'installation pour éliminer le blindage.

Il existe donc toujours un besoin de proposer des matériaux ou dispositifs permettant de conserver un état de surface correct et de limiter les effets de la corrosion pour diminuer et supprimer ce phénomène de blindage.

La présente invention propose pour remédier à ce problème d'utiliser pour la fabrication des parois en contact avec la solution d'acide adipique un matériau choisi dans des nuances d'aciers inoxydables particulières.

A cet effet, l'invention propose une installation ou un cristallisoir pour la cristallisation de l'acide adipique comprenant une cuve ou un cristallisoir, des moyens d'agitation et des moyens pour refroidir et/ou concentrer la solution d'acide adipique, caractérisée en ce qu'au moins une partie des parois en contact avec la solution d'acide adipique et faisant partie de la cuve ou cristallisoir et/ou des moyens pour concentrer et/ou refroidir la solution est réalisée en acier inoxydable austénitique de type AISI 310L selon la nomenclature AISI (USA).

L'acier inoxydable austénitique de type AISI 310L est également désigné X1CrNi25-21 (1.4335) selon la nomenclature européenne.

Selon une caractéristique de l'invention, les moyens pour refroidir et/ou concentrer la solution d'acide adipique sont constitués par des dispositifs en contact avec la solution. Plus particulièrement, des dispositifs comportant une circulation de fluide caloporteur peuvent être avantageusement utilisés pour refroidir la solution. Selon l'invention les parois de ces dispositifs de refroidissement en contact avec la solution d'acide adipique sont réalisées en acier inoxydable austénitique de type AISI 310L. Dans un autre mode de réalisation du procédé de cristallisation de l'acide adipique avec concentration de la solution par évaporation obtenue par mise sous pression réduite de l'installation, les parties de l'installation telles que les parois internes du cristallisoir, par exemple, sont réalisées en acier inoxydable austénitique de type AISI 310L.

Selon une autre caractéristique de l'invention, les surfaces ou parois réalisées en acier inoxydable austénitique de type AISI 310L sont soumises à une opération de polissage avant leur installation dans le cristallisoir. Ce polissage peut être réalisé par tout moyen connu mettant en oeuvre des procédés physiques et/ou chimiques pour diminuer la rugosité de la surface.

A titre indicatif et sans caractère limitatif, il est avantageux que la rugosité de la surface soit inférieure à 0,3µm, mesurée selon la méthode définie par les normes NF EN ISO 3274 et NF EN ISO 4288.

Les moyens pour refroidir et/ou concentrer la solution d'acide adipique peuvent être de forme variée tels que des serpentins, des plaques à double paroi comprenant une circulation de fluides ou analogues.

Le cristallisoir de l'invention convient notamment pour cristalliser l'acide adipique à partir des solutions obtenues à la sortie de l'étape d'oxydation nitrique du mélange cyclohexanone/ cyclohexanol.

L'invention sera mieux illustrée au vu des exemples donnés ci-dessous uniquement à titre indicatif.

Des essais pour déterminer la tenue à la corrosion et l'évolution de l'état de surface d'articles réalisés en différentes nuances d'aciers inoxydables ont été réalisés selon le mode opératoire ci-dessous :
Des éprouvettes de forme parallélipédique de dimensions 50x30 mm dont la surface a été polie pour avoir une rugosité initiale Ra inférieure à 0,1 µm ont été immergées dans un milieu issu de l'oxydation nitrique d'un mélange cyclohexanone/cyclohexanol comprenant une concentration pondérale en acide adipique de 24% et une teneur en acide nitrique de l'ordre de 28% en poids.

La solution est maintenue à une température de 90°C sous pression atmosphérique et est mise sous agitation pendant toute la durée d'immersion. Après 400 heures d'immersion, l'état de surface des éprouvettes et la perte d'épaisseur sont déterminées. Ces éprouvettes sont de nouveau immergées pour une nouvelle durée de 400 heures dans le même milieu. Toutefois, la solution est renouvelée avant chaque nouvelle immersion.

Les éprouvettes testées ont été réalisées en deux nuances d'acier inoxydable :
Eprouvette 1 : acier de type AISI 304L
Eprouvette 2 : acier de type AISI 310L

La composition de ces nuances d'acier est donnée dans le tableau I ci-dessous :

| **metal composition** | **304L** | **310L** |
|---|---|---|
| | **% poids** | |
| C | 0,015 | 0,017 |
| S | 0,002 | 0,006 |
| P | 0,025 | 0,019 |
| Si | 0,248 | 0,14 |
| Mn | 1,690 | 0,593 |
| Cr | 18,410 | 24,29 |
| Ni | 10,480 | 21,62 |
| Mo | 0,125 | 0,317 |
| Cu | 0,069 | 0,167 |
| N | - | - |
| Fe | bal. | bal. |

| | | |
|---|---|---|
| (bal. Signifie complément à 100%) | | |

Les résultats observés sont rassemblés dans le tableau II ci-dessous :

| Eprouvette | | 1 | 2 |
|---|---|---|---|
| Initial | Rugosité Ra (µm) | <0,1 | <0,1 |
| | Perte d'épaisseur (µm/an) | 0 | 0 |
| 400 heures | Rugosité Ra (µm) | 0,5 | < 0,1 |
| | Perte d'épaisseur (µm/an) | 90 | < 5 |
| 800 heures | Rugosité Ra (µm) | 1,9 | 0,2 |
| | Perte d'épaisseur (µm/an) | 130 | < 20 |
| 1200 heures | Rugosité Ra (µm) | 2,8 | 0,2 |
| | Perte d'épaisseur (µm/an) | 130 | < 5 |
| 1600 heures | Rugosité Ra (µm) | 4,1 | 0,3 |
| | Perte d'épaisseur (µm/an) | 230 | < 20 |
| 2000 heures | Rugosité Ra (µm) | 4,7 | 0,3 |
| | Perte d'épaisseur (µm/an) | 90 | < 5 |
| 2400 heures | Rugosité Ra (µm) | 4,7 | 0,3 |
| | Perte d'épaisseur (µm/an) | 140 | < 5 |

Il ressort des résultats ci-dessus que l'éprouvette 2, qui correspond à l'invention, présente une rugosité qui varie très peu au cours du temps comparée à celle mesurée pour un acier de type 304L (éprouvette 1). Cette caractéristique illustre le fait que l'utilisation d'un acier du type 310L selon l'invention permet de conserver un bon état de surface au cours du temps pour la réaction concernée et donc de limiter au maximum le phénomène de blindage.

Par ailleurs, les résultats ci-dessus montrent que la résistance à la corrosion (perte d'épaisseur) pour un acier du type 310L dans le milieu issu de l'oxydation nitrique d'un mélange cyclohexanone/ cyclohexanol (selon l'invention) est accrue.

## Revendications

1. Installation de cristallisation d'acide adipique comprenant une cuve de cristallisation équipée avec des moyens d'agitation, des moyens de refroidissement et/ou concentration de la solution d'acide adipique, **caractérisée en ce qu'**au moins une partie des parois de la cuve de cristallisation et/ou des moyens de refroidissement et/ou concentration en contact avec la solution d'acide adipique est réalisée en un matériau choisi parmi les aciers inoxydables austénitiques de référence de type AISI 310L, selon la nomenclature AISI ou X1CrNi25-21/1.4335 selon la nomenclature européenne.

2. Installation selon la revendication 1, **caractérisée en ce que** la solution d'acide adipique est la solution obtenue par oxydation nitrique d'un mélange cyclohexanone/cyclohexanol.

3. Installation selon l'une des revendications précédentes **caractérisée en ce qu'**elle comprend des dispositifs de refroidissement de la solution comportant une circulation d'un fluide caloporteur, les dits dispositifs étant réalisés en acier inoxydable austénitique de référence de type AISI 310L.

4. Installation selon l'une des revendications précédentes, **caractérisée en ce qu'**au moins une partie des parois interne de la cuve de cristallisation est réalisée en acier inoxydable austénitique de référence de type AISI 310L.

5. Installation selon l'une des revendications précédentes, **caractérisée en ce que** la concentration de la solution est obtenue par mise sous pression réduite de la cuve de cristallisation.

6. Installation selon l'une des revendications précédentes en ce que la surface des parois des dispositifs de l'invention réalisées en acier inoxydable austénitique de type AISI 310L est polie.

7. Installation selon la revendication 6, **caractérisée en ce que** les surfaces polies réalisées en acier inoxydable austénitique de type AISI 310L présentent une rugosité inférieure à 0,3µm, mesurée selon la méthode définie par les normes NF EN ISO 3274 et NF EN ISO 4288.

## Patentansprüche

1. Einrichtung zum Kristallisieren von Adipinsäure, die einen Kristallisationsbehälter umfasst, der mit Rührmitteln, Mitteln zum Kühlen und/oder Konzentrieren der Adipinsäurelösung versehen ist, **dadurch gekennzeichnet, dass** zumindest ein Abschnitt der Wände des Kristallisationsbehälters und/oder der Kühl- und/oder Konzentrationsmittel im Kontakt mit der Adipinsäurelösung aus einem Werkstoff gefertigt ist, der ausgewählt ist aus austenitischen rostfreien Bezugsstählen vom Typ AISI 310L, gemäß der AISI-Nomenklatur, oder X1CrNi25-21/1.4335 gemäß der europäischen Nomenklatur.

2. Einrichtung nach Anspruch 1, **dadurch gekennzeichnet, dass** die Adipinsäurelösung die Lösung ist, die durch Oxidation mit Salpetersäure einer Mischung Cyclohexanon/Cyclohexanol erhalten wird.

3. Einrichtung nach einem der vorhergehenden Ansprüche, **dadurch gekennzeichnet, dass** sie Vorrichtungen zum Kühlen der Lösung umfasst, die einen Kreislauf eines Wärmeträgerfluids aufweisen, wobei die Vorrichtungen aus austenitischem rostfreiem Bezugsstahl vom Typ AISI 310L gefertigt sind.

4. Einrichtung nach einem der vorhergehenden Ansprüche, **dadurch gekennzeichnet, dass** zumindest ein Abschnitt der Innenwände des Kristallisationsbehälters aus austenitischem rostfreiem Bezugsstahl vom Typ AISI 310L gefertigt ist.

5. Einrichtung nach einem der vorhergehenden Ansprüche, **dadurch gekennzeichnet, dass** die Konzentration der Lösung erhalten wird, indem der Kristallisationsbehälter einem verminderten Druck ausgesetzt wird.

6. Einrichtung nach einem der vorhergehenden Ansprüche, **dadurch gekennzeichnet, dass** die Oberfläche der aus austenitischem rostfreiem Stahl vom Typ AISI 310L gefertigten Wände der Vorrichtungen der Erfindung poliert ist.

7. Einrichtung nach Anspruch 6, **dadurch gekennzeichnet, dass** die aus austenitischem rostfreiem Stahl vom Typ AISI 310L gefertigten polierten Oberflächen eine nach dem in der Norm NF EN ISO 3274 und NF EN ISO 4288 definierten Verfahren gemessene Rauheit von unter 0,3 µm aufweisen.

## Claims

1. Plant for the crystallization of adipic acid comprising a crystallization vessel equipped with means for stirring and means for cooling and/or concentrating the adipic acid solution, **characterized in that** at least a part of the walls of the crystallization vessel and/or of the means for cooling and/or concentrating in contact with the adipic acid solution is made of a material chosen from reference austenitic stainless steels of AISI 310L type, according to the AISI nomenclature, or X1CrNi25-21/1.4335 type, according to the European nomenclature.

2. Plant according to Claim 1, **characterized in that** the adipic acid solution is the solution obtained by oxidation of a cyclohexanone/cyclohexanol mixture by nitric acid.

3. Plant according to either of the preceding claims, **characterized in that** it comprises devices for cooling the solution comprising circulation of a heat-exchange fluid, the said devices being made of reference austenitic stainless steel of AISI 310L type.

4. Plant according to one of the preceding claims, **characterized in that** at least a part of the internal walls of the crystallization vessel is made of reference austenitic stainless steel of AISI 310L type.

5. Plant according to one of the preceding claims, **characterized in that** the concentration of the solution is obtained by placing the crystallization vessel under reduced pressure.

6. Plant according to one of the preceding claims, **characterized in that** the surface of the walls of the devices of the invention made of austenitic stainless steel of AISI 310L type is polished.

7. Plant according to Claim 6, **characterized in that** the polished surfaces made of austenitic stainless steel of AISI 310L type exhibit a roughness of less than 0.3 µm, measured according to the method defined by Standards NF EN ISO 3274 and NF EN ISO 4288.
